# EUROPEAN PATENT SPECIFICATION

(11) **EP 1 019 095 B1**
(45) Date of publication and mention of the grant of the patent: **16.01.2002**
(21) Application number: 98918501.2
(22) Date of filing: 21.04.1998
(51) Int. Cl.: A61K 51/04, C01G 45/04, C07F 13/00

(54) **METHOD FOR THE PREPARATION OF FACIAL METAL TRICARBONYL COMPOUNDS AND THEIR USE IN THE LABELLING OF BIOLOGICALLY ACTIVE SUBSTRATES**
VERFAHREN ZUR HERSTELLUNG VON FACIAL METALL TRICARBONYL VERBINDUNGEN UND IHRE ANWENDUNG ZUR MARKIERUNG VON BIOSUBSTRATEN
PROCEDE DE PREPARATION DE COMPOSES CARBONYLE-METAL FACIAUX ET LEUR UTILISATION DANS LE MARQUAGE DE SUBSTRATS BIOLOGIQUEMENT ACTIFS

(30) Priority: 25.04.1997 EP 97201232
(43) Date of publication of application: 19.07.2000
(73) Proprietor: Mallinckrodt Inc., St. Louis, MO 63134 (US)
(72) Inventor: ALBERTO, Roger, CH-8400 Winterthur (CH); SCHIBLI, Roger, CH-5232 Villingen PSI (CH); EGLI, André, CH-8712 Stäfa (CH)
(74) Representative: Van Someren, Petronella F. H. M.
(86) International application number: US9807979
(87) International publication number: WO9848848

(56) References cited:
- EP-A- 0 105 785
- BECK, WOLFGANG ET AL: "Metal complexes with biologically important ligands. XVIII. Histidinato-carbonyl complexes of molybdenum and tungsten" J. ORGANOMET. CHEM. (1980), 191(1), 73-7 CODEN: JORCAI;ISSN: 0022-328X, 1980, pages 73-77, XP002043518
- BAMFORD, CLEMENT H. ET AL: "Evidence for the formation of the triaquatricarbonylmanganese(I) cation and related derivatives from pentacarbonylchloromanganese" J. CHEM. SOC., DALTON TRANS. (1978), (1), 4-8 CODEN: JCDTBI;ISSN: 0300-9246, 1978, pages 4-8, XP002043519
- VERONA, IVANA ET AL: "Regioselectivity in the nucleophilic functionalization of dibenzofuran, dibenzothiophene and xanthene complexes of Mn(CO)3+" J. ORGANOMET. CHEM. (1996), 524(1-2), 71-80 CODEN: JORCAI;ISSN: 0022-328X, 1996, XP002043520
- MEDER, HANS JOCHEN ET AL: "Metal complexes with biologically important ligands. XLII. Carbonyl metal complexes with anions of polyfunctional.alpha.-amino acids" Z. NATURFORSCH., B: ANORG. CHEM., ORG. CHEM. (1986), 41B(10), 1247-54 CODEN: ZNBAD2;ISSN: 0340-5087, 1986, XP002043521
- CHEMICAL ABSTRACTS, vol. 126, no. 18, 5 May 1997 Columbus, Ohio, US; abstract no. 245901, EGLI, ANDRE ET AL: "Hydrolysis of the Organometallic Aqua Ion fac- Triaquatricarbonylrhenium(I). Mechanism, pKa, and Formation Constants of the Polynuclear Hydrolysis Products" XP002043522 & ORGANOMETALLICS (1997), 16(9), 1833-1840 CODEN: ORGND7;ISSN: 0276-7333, 1997,
- SCHBIGER P.A. ET AL.: "Versatilty of the "fac-Tc(CO)3" (Tc-99m) moiety for the labeling of various biomolecules." JOURNAL OF NUCLEAR MEDICINE, ABSTRACT BOOK, May 1997, NEW YORK US, page 180P XP002068168

## Description

The invention relates to a method of preparation of facial metal tricarbonyl compounds and further co-ordinated facial metal tricarbonyl compounds. The invention further relates to the use of said facial metal tricarbonyl compounds in the labelling of biologically. active substrates and other ligands, and to a kit for preparing a facial metal tricarbonyl compound or further co-ordinated facial metal tricarbonyl compounds.

The application of metal complexes, with a wide variety of radionuclides, in the field of nuclear medicine has become a major tool in diagnosis and also more recently in therapy. The metal complexes are often attached to a biologically active substrate that acts as a targeting agent. One of the most widely applied procedures for the metal-labelling of biologically active substrates such as proteins, peptides, sugars or small biologically active compounds consists in stabilizing the M(V)=O moiety of (radioactive) metals of group 7B of the periodic table with different tetradentate ligands. After reduction, the M(V)=O moiety is intermediately stabilized with a larger amount of an auxiliary ligand such as glucoheptonate which is subsequently substituted by the chelator attached to the system to be labelled. This method has proven to be successful in many cases but suffers from some major disadvantages such as the required high denticity and the bulkiness of the ligand and the difficulty in synthesizing and attaching such ligand.

It is known in the art (Alberto et al., J. Nucl. Biol. and Med. 1994, 38, 388-90) that facial metal tricarbonyl complexes of radioactive metals of group 7B of the periodic table are very convenient starting materials for substitution reactions in organic solvents as well as in water, as these compounds are stable in water for weeks, even if exposed to air. Therefore said compounds would be very useful for the labelling of biologically active substrates, such as amino acids, peptides,. proteins, sugars and any receptor binding molecules. A major drawback, however, of these compounds until now is that they have only been available from high temperature carbonylation reactions and with the aid of the pyrophoric and toxic and therefore dangerous reducing agent BH₃ (Alberto et al., Low CO pressure synthesis of (NEt)₂[MX₃(CO)₃] (M = Tc, Re) and its Substitution Behaviour in Water and Organic Solvents. Technetium in Chemistry and Nuclear Medicine, No 4, Cortina International, Milano, 1994).

It is the objective of the present invention to provide for a method of preparing facial metal tricarbonyl compounds of (radioactive) metals of group 7B with the aid of easily available and low-toxic starting materials at moderate temperature and at normal pressure of CO, in a reasonable time and with high yield.
Such a method would be a powerful tool that can be used for the synthesis of diagnostic and therapeutic agents, especially for the synthesis of said diagnostic and therapeutic agents derived from radioactive metals with a short lifetime, in order to have access to these labelled compounds in poorly-equipped hospital laboratories. When the above mentioned diagnostic agent is labelled with a radionudide it can be detected by the so-called single photon emission computerized tomography (SPECT and SPET), when it is labelled with a paramagnetic metal atom it can be detected by magnetic resonance imaging.

The above-defined objective can be achieved, according to the present invention, by a method of preparing a compound of the general formula

*fac*-[M(CO)₃(OH₂)₃]⁺ (I)

wherein M is Mn, ^{99m}Tc, ¹⁸⁶Re or ¹⁸⁸Re,
by reacting a metal in the permetallate form (MO₄⁻ form) with carbon monoxide and a reducing agent, characterized in that a mixture of a base, a reducing agent soluble in water but not substantially decomposed by water, and optionally a stabilizing agent is solved in a water containing solvent system containing a solution of the metal in the permanganate, pertechnetate or perrhenate form in the presence of carbon monoxide.
The metal M is preferably ^{99m}Tc, ¹⁸⁶Re or ¹⁸⁸Re, as these radionuclides, when used in diagnostic or therapeutic agents, have the advantage that they can be applied in very low concentrations, which minimizes the risk of toxicity.

The term "not substantially decomposed by water" means that upon the addition of the solution of permanganate, pertechnetate or perrhenate in water, the velocity of the decomposition reaction of the reducing agent with water is zero or very low compared with the reaction of said reducing agent with the permanganate, pertechnetate or perrhenate, so that the reaction with said permetallate is completed when still enough of the reducing agent is present.
It is very surprising that a quantitative reduction of permetallates in water containing solvent systems can be achieved at moderate temperature and in reasonable times with reducing agents that are nucleophilic and that are generally considered as less reactive than the electrophilic reducing agent BH₃ known in the art.

The method of the invention can be easily performed just by mixing the permetallate solution with the other reagents in the presence of carbon. monoxide. The permetallate solution may optionally contain halide ions needed for the elution of the permetallate from a generator. The carbon monoxide may be supplied by using a closed system with an atmosphere containing a sufficient amount of carbon monoxide, or. by flushing the carbon monoxide gas through the solution.
The base used is preferably an inorganic base, selected from the group of stable hydroxides and carbonate salts such as NaOH, KOH, NaHCO₃, Na₂CO₃, KHCO₃, K₂CO₃, Ca(OH)₂ and Mg(OH)₂. Most preferred is Na₂CO₃. The base is added in a molar ratio to the reducing agent of between 0.1 and 2, and preferably in a molar ratio of approx. 0.35.
The reaction can be performed with and without a stabilizing agent. As a stabilizing agent gentisate (2,5-dihydroxybenzoate), glucoheptonate, citrate or tartrate can be used. The preferred stabilizing agent is tartrate, e.g. as NaKtartrate. The stabilizing agent is added to the reaction mixture in such an amount that its concentration is higher than that of the metal to be reduced.
For the reduction several reducing agents can be used, such as borohydride anion (BH₄⁻) or substituted borohydride anion wherein up to three of the hydrogen atoms which comprise the borohydride anion have been independently replaced by inert substituents. of said inert substituents are alkoxy or alkylcarbonyloxy groups containing 1 to 10 carbon atoms and cyano groups. The counterion of the reducing group may consist of a metal of group 1A or 2A of the periodic table or zinc or an ammonium or tetrasubstituted ammonium or tetrasubstituted phosphonium ion, wherein the four substituents are each independently alkyl groups containing from 1 to 10 carbon atoms, hydroxyalkyl groups or alkoxyalkyl groups containing from 2 to 10 carbon atoms or aryl groups,

Preferred reduction reagent is borohydride anion, especially in the form of compounds such as sodium borohydride, potassium borohydride, lithium borohydride and zinc borohydride. Most preferred reducing agent is sodium borohydride.
The reducing agent is reacted with the permetallate in a molar ratio higher than 3. The reduction reaction can be performed at a temperature between 20°C and 100°C. The preferred reaction temperature is approx. 75°C. The heating of the reaction mixture can be performed in the normal way but also by micro-wave heating. The reaction can also be performed by the application of ultra sound, e.g. by carrying out the reactions in an ultrasonic bath at room temperature, normally leading to the same reaction rate at lower reaction temperature.

The compound of the general formula (I) obtained is very suitable for the labeling of biologically active substrates, such as amino acids, peptides, proteins, sugars, small receptor binding molecules or cells.
Examples of peptides that may be labelled are growth factors, somatostatin, bombesin, insulin; LHRH, gastrin, gastrin releasing peptide, thyrotropin releasing hormone, thyroid stimulating hormone, prolactin, vasoactive intestinal peptide (VIP), pituitary adenylate cyclase-activating polypeptide (PACAP), angiotensin, neurotensin, interferons, IL-1, IL-4 and IL-6, monoclonal antibodies and their analogues and derivatives. After labelling with a suitable labelling substance these peptides can e.g. be used in the detection and localisation or treatment of malignant human tumours.

Examples of sugars that may be labelled are glucose and deoxyglucose and derivatives of said compounds.

Small receptor binding molecules are defined as non-peptide molecules which are binding to a receptor and normally have a molecular mass below approximately 500 Daltons.
Examples of small receptor binding molecules that may be labelled are substances for the serotonergic system as described in WO 96/30054, or substances for the dopaminergic system (e.g. raclopride, β-CIT, lisuride), for the cholinergic system (e.g. epibatidine), for the glutaminergic system (e.g. mematine) or for the benzodiazepine system (e.g, flumazenil, iomazenil). Examples of metabolic active molecules that may be labelled are DOPA, Tyrosine, mlBG, MAO-I and analogues thereof.

Examples of cells that may be labelled are red and white blood cells.

As a result of the labeling of (biologically active) substrates with a compound of the general formula I, a further coordinated compound of the general formula

*fac*-[M(CO)₃(X)₂L₁]ⁿ (II),

*fac*-[M(CO)₃(X)L₂]ⁿ (III)

or

*fac*-[M(CO)₃L₃]ⁿ (IV),

wherein:
- M: is Mn, ^{99m}Tc, ¹⁸⁶Re or ¹⁸⁸Re;
- L₁: is a monodentate ligand,
- L₂: is selected from the group consisting of a bidentate ligand and two monodentate ligands, and
- L₃: is selected from the group consisting of a tridentate ligand, a monodentate ligand and a bidentate ligand, and three monodentate ligands;
- X: is H₂O or a halide ion;
- n: the sum of the charge of the ligands L₁ or L₂ or L₃ and X increased with one + charge
is obtained.
After the labeling reaction the ligand X is usually H₂O. One of the H₂O ligands may, however, be replaced by a halide ion, when available, to neutralize the charge of the complex. This is often the case for compounds of the general formula III.
When the ligand L₁, L₂ or L₃ before and/or after labeling with the facial metal tricarbonyl compound is the biologically active molecule, the present invention gives easy access to compounds that directly can be used as a diagnostic and therapeutic agent.

Examples of monodentate ligands within the definition of L₁, L₂ and L₃ are (biologically active) substrates bearing groups such as phosphines, isonitriles, nitriles, imidazoles, thioethers and pyridine-like aromatic amines.
Examples of bidentate ligands within the definition of L₂ and L₃ are (biologically active) substrates bearing pyridin, imidazole or pyrazole groups, such as histidine, histamine, functionalized imidazole systems, bidentate thioethers, bidentate isocyanides, Schiff-base type ligands and picolinic acid.

Examples of tridentate ligands within the definition of L₃ are tris-pyrazolyl borate, tris-pyrazolylmethane, tris-imidazolyl borate, tris-pyrazolylmethane, 1,4,7-trithiacyclononane (9-aneS₃) and triazacyclononane (9-aneN₃), histidine, methionine, cystein derivatized at the thiol group to give a thioether and cyclopentadienyl derivatives.

In some cases it may be advantageous to prepare the radiolabelled bioactive compound in one step. This objective can be achieved according to the present invention, with a method of preparing a compound of the general formula

*fac*-[M(CO)₃(X)₂L₁]ⁿ (II),

*fac*-[M(CO)₃(X)L₂]ⁿ (III)

or

*fac*-[M(CO)₃L₃]ⁿ (IV),

wherein:
- M: is Mn, ^{99m}Tc, ¹⁸⁶Re or ¹⁸⁸Re;
- L₁: is a monodentate ligand,
- L₂: is selected from the group consisting of a bidentate ligand and two monodentate ligands, and
- L₃: is selected from the group consisting of a tridentate ligand, a monodentate ligand and 'a bidentate ligand, and three monodentate ligands;
- X: is H₂O or a halide ion;
- n: the sum of the charge of the ligands L₁ or L₂ or L₃ and X increased with one + charge;
characterized in that a mixture of a base, ligands L₁ or L₂ or L₃, a reducing agent soluble in water but not substantially decomposed by water, and optionally a stabilizing agent is solved in a water containing solvent system containing a solution of the metal in the permanganate, pertechnetate or perrhenate form in the presence of carbon monoxide and optionally in the presence of halide.

Especially in the case of radiolabelled compounds it is frequently impossible to put the ready-for-use composition at the disposal of the user, in connection with the often poor shelf life of the radiolabelled compound and/or the short half-life of the radionuclide used. In such cases the user will carry out the labelling reaction with the 'metal in the clinical hospital or laboratory. For this purpose the various reaction ingredients are then offered to the user in the form of a so-called "kit". It will be obvious that the manipulations necessary to perform the desired reaction should be as simple as possible to enable the user to prepare from the kit the radioactive labelled composition by using the facilities that are at his disposal. Therefore the invention also relates to a kit for preparing a labelling composition, which labelling composition contains compound of formula I as the labelling agent.
Such a kit for the labelling of a biologically active substrate, according to the present invention, comprises (i) a reducing agent soluble in water but not substantially decomposed by water, (ii) a base, (iii) if desired, a stabilizing agent and/or a chelator and (iv) if desired one or more inert pharmaceutically acceptable carriers and/or formulating agents and/or adjuvants, at least one of said ingredients (i) to (iv) being stored in a container having an atmosphere containing a sufficient amount of carbon monoxide, said ingredients (i) to (iv) optionally independently being combined, and (v) instructions for use with a prescription for reacting the ingredients of the kit with a metal selected from the group consisting of Mn, ^{99m}Tc, ¹⁸⁶Re or ¹⁸⁸Re in the form of a permetallate solution.

It is the merit of the present invention, disclosing an easy way of preparing facial tricarbonyl metal compounds within a time-frame that is reasonable compared with the half-life time of the radioactive isotopes involved, and with high yields, that a kit can be prepared for the labelling of biologically active substrates with said facial tricarbonyl metal compounds.

In some cases it may be advantageous to enclose a bioactive substrate in the kit so that a kit is obtained for the preparation of a radiopharmaceutical composition.

Alternatively the biologically active compound is formed upon the reaction of the ligand with the facial metal tricarbonyl compound.
Such a kit for the preparation of a diagnostic and therapeutic pharmaceutical composition, according to a different embodiment of the present invention, comprises (i) a suitable substrate to be labelled with a metal selected from the group consisting of Mn, ^{99m}Tc, ¹⁸⁶Re or ¹⁸⁸Re, (ii) a reducing agent soluble in water but not substantially decomposed by water, (iii) a base, (iv) if desired, a stabilizing agent and/or a chelator, (v) if desired one or more inert pharmaceutically acceptable carriers and/or formulating agents and/or adjuvants, at least one of said ingredients (i) to (v) being stored in a container having an atmosphere containing a sufficient amount of carbon monoxide, said ingredients (i) to (v) optionally independently being combined, and (vi) instructions for use with a prescription for reacting the ingredients of the kit with said metal in the form of a permetallate solution.

The preparation of the diagnostic and therapeutic pharmaceutical composition with the aid of the above mentioned kit enclosing a (biologically active) substrate can take place in two alternative embodiments. In the first embodiment the facial tricarbonyl metal compound is prepared first and then reacted with the substrate to be labelled. In the second embodiment the reduction step is carried out in the presence of the substrate to be labelled, directly leading to the labelled compound.

The invention will now be described in greater detail with reference to the following specific Examples.

### Example 1. Synthesis of [^{99m}Tc(OH₂)₃(CO)₃]⁺ from aqueous solution

In a 10 ml closable vial the following chemicals are put together: 5.5 mg of NaBH₄, 4.0 mg Na₂CO₃ and 20.0 mg NaKtartrate. The vial is closed with a serum stopper and flushed for 10 minutes with carbon monoxide gas with the aid of a syringe. 3 ml of a 0.9% NaCl solution from a Mo-99/Tc-99m generator, having an activity of about 100 mCi, is added via the septum and the vial is heated to 75°C during 30 minutes and then cooled to room temperature. The product is analysed by TLC on standard Merck silica gel plates with methanol/conc. HCl = 99/1 as mobile phase followed by analysis of the silica gel plate by means of a radioactivity scanner. The yield of the reduction of pertechnetate to facial [^{99m}Tc(OH₂)₃(CO)₃]⁺ is > 95% according to TLC. After neutralizing the solution with a solution of PBS (phosphate buffer (pH = 7.4, saline 0.9%)) a neutral physiological solution, suitable for labelling is obtained.
Table 1 shows that solutions of [^{99m}Tc(OH₂)₃(CO)₃]⁺ having an activity up to 700 mCi can be obtained under different reaction conditions.

**Table 1.**

| **Preparation of [**^{**99m**}**Tc(OH**_{**2**}**)**_{**3**}**(CO)**_{**3**}**]**^{**+**} **under different reaction conditions.** | | | | | | | |
|---|---|---|---|---|---|---|---|
| Exp. | Stabilizing agent | Volume TcO₄⁻ sol. (ml) | Activity (mCi) | Solvent | Temp. (°C) | React. time (min) | Yield (TLC) (%) |
| 1 | NaKtartrate | 3 | ≈ 100 | H₂O | 75 | 30 | >95 |
| 2 | NaKtartrate | 3 | ≈ 400 | H₂O | 75 | 30 | > 95 |
| 3 | NaKtartrate | 3 | ≈ 700** | H₂O | 75 | 30 | >95 |
| 4 | NaKtartrate | 3 | n.d.* | H₂O | 75 | 30 | >95 |
| 5 | NaKtartrate | 6 | n.d.* | H₂O | 75 | 30 | >95 |
| 6*** | NaKtartrate | 3 | n.d.* | H₂O | 75 | 30 | 40 |
| 7 | - | 3 | n.d.* | H₂O | 75 | 30 | 70 |
| 8 | Nacitrate | 3 | n.d.* | H₂O | 75 | 30 | 20 |
| 9 | Naformiate | 3 | n.d.* | H₂O | 75 | 30 | 35 |
| 10 | NaKtartrate | 3 | n.d.* | H₂O/EtOH 80/20 | 75 | 30 | >95 |
| 11 | NaKtartrate | 3 | n.d.* | H₂O | 100 | 10 | 80 |

| | | | | | | | |
|---|---|---|---|---|---|---|---|
| * Activity not determined exactly, but always between 50 and 200 mCi. | | | | | | | |
| ** Activity determined after dilution to 1% | | | | | | | |
| *** 4.0 mg Ca(OH)₂ has bean used as a base instead of 4.0 mg Na₂CO₃ | | | | | | | |

### Example 2. Synthesis of complexes of composition [^{99m}TcL(CO)₃]

### 2.1 Synthesis of [^{99m}Tc(his)(CO)₃] via [^{99m}Tc(OH₂)₃(CO)₃]⁺.

After completion of the reaction as described in Example 1, 0.1 µmol of histidine is added to the solution of pH 7.4. According to TLC the reaction is complete after 1 hour.
When 0.01 µmol of histidine is added at room temperature, the reaction takes 5-10 hours before completion; the reaction is completed in less than 1 hour at 70°C.

### 2.2 Direct synthesis of [^{99m}Tc(his)(CO)₃].

The experiment is carried out as described in Example 1. Concerted to the addition of the generator eluate to the cold vial, 0.03 µmol of histidine is added to said cold vial. After heating during 30 minutes [^{99m}Tc(his)(CO)₃]⁺ is obtained in almost quantitative yield according to TLC.

### 2.3 Synthesis of [^{99m}Tc((lys-gly-(his)₅)(CO)₃]⁺ via [^{99m}Tc(OH₂)₃(CO)₃]⁺.

After completion of the reaction as described in Example 1, 500 pmol of the octapeptide lys-gly-gly-(his)₅ is added to the solution. According to TLC the reaction is complete after 1 hour at room temperature.
When 300 pmol of lys-gly-gly-(his)₅ is added the reaction takes 3 hours to complete.

### 2.4 Summary of preparation of further complexes of composition [^{99m}TcL(CO)₃]

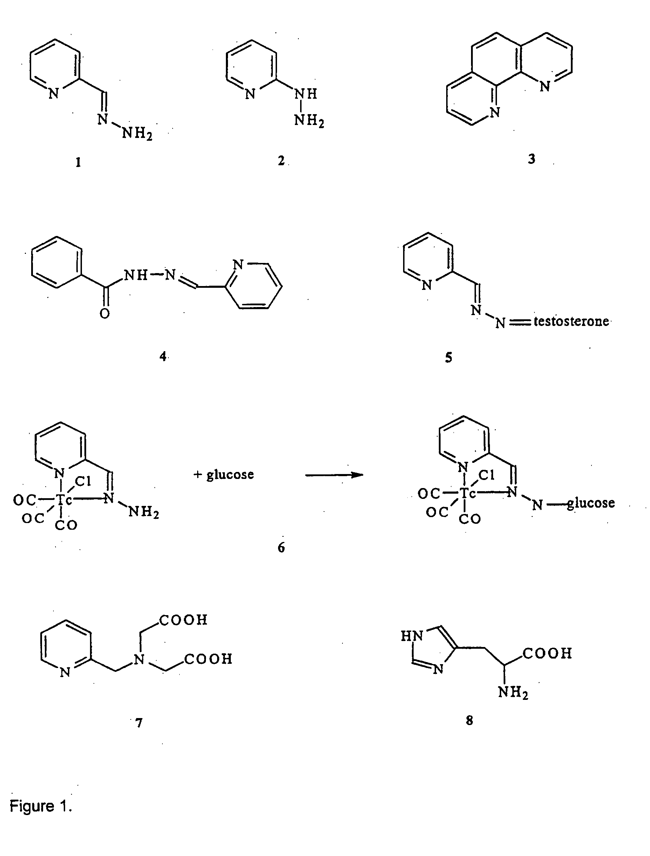

The [^{99m}Tc(OH₂)₃(CO)₃]⁺, is prepared as described in Example 1 and neutralized. The ligand (see Figure 1) is added with subsequent complexation time and temperature and in amount and concentration as indicated in Table 2. Table 2 also indicates the yields obtained as determined by TLC analysis as described in Example 1 and the possibility of carrying out the reaction in a one pot process.

**Table 2.**

| **Preparation of [**^{**99m**}**TcL(CO)**_{**3**}**] with different ligands and under different reaction conditions.** | | | | | | | |
|---|---|---|---|---|---|---|---|
| Ligand (Fig.1) | pH. | time | temp | Conc. | absolute amount | yield | one pot **** |
| 1 | PBS | 30' | 70°C | 3 µM | 5 nmol | 92 % | partially |
| 2 | PBS | 2-3 h | 37°C | 20 µM | 10 nmol | 74 % | yes |
| 3 | PBS | 4 h | 37°C | 100 µM | 50 nmol | 89% | yes |
| 4 | PBS | 6 h | 37°C | 100 µM | 50 nmol | 72 % | - |
| 5 | PBS/ CH₃OH | 1h | 50°C | 100 µM | 100 nmol | 65 % | - |
| 6 | PBS | 1h+ 1h | 50°C | * | | 97%+ 30% | - |
| 7*** | PBS | 30' | 75°C | 25 µM | 25 nmol | 95% | yes |
| 7*** | PBS | 15' | ** | 25 µM | 25 nmol | 95% | yes |
| 8 | PBS | 1h | 75°C | 10 µM | 2 nmol | 95% | yes |

| | | | | | | | |
|---|---|---|---|---|---|---|---|
| * 50µl 10⁻³M ligand 1, then 50µl 10⁻²M glucose | | | | | | | |
| ** 15 min ultra sound r.t. | | | | | | | |
| *** After the reaction the product was stored for 23 h at 37°C and appeared to be stable | | | | | | | |
| **** possibility to perform as a one pot synthesis | | | | | | | |

From the compound derived from ligand 5 (see structure below) the ¹H-NMR spectrum was determined before and after complexation with "cold" [Tc(OH₂)₃(CO)₃]⁺. A: aromatic region of NMR spectrum before complexation: ¹H-NMR (CDCl₃)[r.t., δ in ppm] = 8.75 (1H, d)(a), 8.36 (1H, s)(e), 8.14 (1H, d)(d), 7.99 (1H, t)(b), 7.55 (1H, m)(c), 6.07 (1H, s)(f). B: aromatic region of NMR spectrum after complexation: ¹H-NMR (CDCl₃)[r.t., δ in ppm] = 9.12 (1H, d)(a), 8.51 (1H, s)(e), 8.37 (1H, t)(b), 8.19 (1H, d)(d), 7.87 (1H, m)(c), 6237 (1H, s)(f).

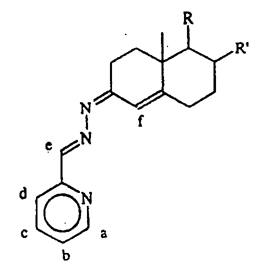

### Example 3. Labelling of antibodies with [^{99m}Tc(OH₂)₃(CO)₃]⁺

### 3.1 Labelling as function of concentration.

Labelling kinetics is tested as a function of Mab concentration. Concentration above 2-3 mg/ml yields quantitative labelling after 2 hours, whereas below 1 mg the yield is about 40-50% according to TLC.

### 3.2. In vitro biological activity of labelled monoclonal antibody 35 (Mab-35)

An amount of [^{99m}Tc(OH₂)(CO)₃]⁺ as prepared in Example 1 is used for labelling of 1.2 mg of Mab-35. After 3 hours of incubation at 37 °C, the Mab is separated over a PD-10 size exclusion gel chromatography column with 38% yield. The labelled Mab is brought to a Lindmo testing (T. Lindmo, P.A. Brunn, Methods in Enzymology 1986, 121, 678), showing 100% biological activity.

### Example 4. Labelling of His-Neurotensin(8-13) with [^{99m}Tc(OH₂)₃(CO)₃]⁺

0.9 ml of [^{99m}Tc(OH₂)₃(CO)₃]⁺ as prepared in example 1 is mixed with 0.1 ml of 10⁻³M His-Neurotensin(8-13) (HRRPYIIL) and kept in a sealed tube at 75°C for one hour. After this time the reaction mixture is cooled to room temperature. As evident from reversed phase column HPLC the yield is >95%. The K_{*d*} of this compound on coloncarcinoma cells HT29 is 1.0 nM.

### Example 5. Labelling of the protein fragment recombinant scFv with 6xHis-tag with [^{99m}Tc(OH₂)₃(CO)₃]⁺

0.1 ml of [^{99m}Tc(OH₂)₃(CO)₃]⁺ as prepared in Example 1 is mixed with 0.1 ml of 1M MES-buffer pH 6.2 and 0.1 ml of 150 µM scFv-6xHis and kept at 37°C for 20 min. After this time the reaction mixture is separated on a Sephadex® G-25 Superfine sizing column. Typical incorporations of ^{99m}Tc are 70% to 84%, with biological activities (measured by the Lindmo testing mentioned in Example 3) ranging from 57% to 90%. K_{*d*} values were not significantly altered by the ^{99m}Tc incorporation: measurement by BlAcore of the unlabeled scFv: 0.5x10⁻⁸M, ^{99m}Tc-labelled scFv: 1x10⁻⁸M, ¹²⁵I-labelled scFv: 4x10⁻⁸M.

### Example 6. Labelling of biotin with [^{99m}Tc(OH₂)₃(CO)₃]⁺

[^{99m}Tc(OH₂)₃(CO)₃]⁺ is prepared as described in example 1. 1.300µl of a 10⁻³M biotin-hydrazide-pyridine solution is added to 2 ml of the Tc-carbonyl compound and is incubated at 50°C for 2 hours to yield a Tc labelled compound with a purity of 50%. The compound is purified over an equilibrated (5 ml MeOH/H₂O=1/1) SepPac column by loading and eluting by-products with 2 ml H₂O and then with 4 ml MeOH/H₂O=1/1 followed by eluting the desired product with 2 ml of MeOH. Final purity of the compound is 98%. Stability control: No decomposition in methanol after 24 hours; 32% decomposition in PBS buffer after 24 hours. Results of binding test to Streptavidin-beads: 0.5 % unspecific binding and 81% specific binding.

## Claims

1. Method of preparing a compound of the general formula
*fac*-[M(CO)₃(OH₂)₃]⁺ (I)
wherein M is Mn, ^{99m}Tc, ¹⁸⁶Re or ¹⁸⁸Re,
by reacting a metal in the permetallate form with carbon monoxide and a reducing agent, **characterized in that** a mixture of a base, a reducing agent soluble in water but not substantially decomposed by water, and optionally a stabilizing agent is solved in a water containing solvent system containing a solution of the metal in the permanganate, pertechnetate or perrhenate form in the presence of carbon monoxide.

2. Method of preparing a compound of the general formula
*fac*-[M(CO)₃(X)₂L₁]ⁿ (II),
*fac*-[M(CO)₃(X)L₂]ⁿ (III)
or
*fac*-[M(CO)₃L₃]ⁿ (IV),
wherein:
M is Mn, ^{99m}Tc, ¹⁸⁶Re or ¹⁸⁸Re;
L₁ is a monodentate ligand,
L₂ is selected from the group consisting of a bidentate ligand and two monodentate ligands, and
L₃ is selected from the group consisting of a tridentate ligand, a monodentate ligand and a bidentate ligand, and three monodentate ligands;
X is H₂O or a halide ion;
n is the sum of the charge of the ligands L₁ or L₂ or L₃ and X increased with one + charge;
**characterized in that** a compound of general formula I prepared as claimed in claim 1 is reacted with ligands L₁ or L₂ or L₃, optionally in the presence of a halide.

3. Method of preparing a compound of the general formula
*fac*-[M(CO)₃(X)₂L₁]ⁿ (II),
*fac*-[M(CO)₃(X)L₂]ⁿ (III)
or
*fac*-[M(CO)₃L₃]ⁿ (IV),
wherein:
M is Mn, ^{99m}Tc, ¹⁸⁶Re or ¹⁸⁸Re;
L₁ is a monodentate ligand,
L₂ is selected from the group consisting of a bidentate ligand and two monodentate ligands, and
L₃ is selected from the group consisting of a tridentate ligand, a monodentate ligand and a bidentate ligand, and three monodentate ligands;
X is H₂O or a halide ion;
n is the sum of the charge of the ligands L₁ or L₂ or L₃ and X increased with one + charge;
**characterized in that** a mixture of a base, ligands L₁ or L₂ or L₃, a reducing agent soluble in water but not substantially decomposed by water, and optionally a stabilizing agent is dissolved in a water containing solvent system containing a solution of the metal in the permanganate, pertechnetate or perrhenate form in the presence of carbon monoxide and optionally in the presence of a halide.

4. Method according to claims 2-3, **characterized in that** the ligands L₁-L₃ are or are derived from biologically active substrates selected from the group consisting of amino acids, peptides, proteins, sugars, small receptor binding molecules and body cells.

5. Method of labeling according to claim 4, **characterized in that** the substrate is an amino acid, a peptide or a protein.

6. Method according to claim 1 or 3, **characterized in that** the base is an inorganic base; preferably selected from the group consisting of NaOH, KOH, NaHCO₃, Na₂CO₃, KHCO₃, K₂CO₃, Ca(OH)₂ and Mg(OH)₂.

7. Method according to claim 1, 3 or 6, **characterized in that** the molar ratio between the base and the reducing agent is between 0.1 and 2, and preferably is approx. 0.35.

8. Method according to claim 1, 3 or 6 - 7, **characterized in that** the reducing agent is selected from the group consisting of borohydride anion and substituted borohydride anion wherein up to three of the hydrogen atoms which comprise the borohydride anion have been replaced by inert substituents.

9. Method according to claim 8, **characterized in that** the reducing agent is a borohydride anion derived from of a salt selected from the group consisting of sodium borohydride, potassium borohydride, lithium borohydride and zinc borohydride.

10. Method according to claim 9, **characterized in that** the reducing agent is sodium borohydride.

11. Method according to claim 10, **characterized in that** the molar ratio of the reducing agent to the permetallate is at least 3.

12. Method according to claims 1, 3 and 6-11, **characterized in that** the reaction time is between 20°C and 100°C and preferably is approx. 75°C.

13. A kit for the preparation of labelling composition, comprising (i) a reducing agent soluble in water but not substantially decomposed by water, (ii) a base, (iii) if desired, a stabilizing agent and/or a chelator and (iv) if desired one or more inert pharmaceutically acceptable carriers and/or formulating agents and/or adjuvants, at least one of said ingredients (i) to (iv) being stored in a container having an atmosphere containing a sufficient amount of carbon monoxide, said ingredients (i) to (iv) optionally independently being combined, and (v) instructions for use with a prescription for reacting the ingredients of the kit with a metal selected from the group consisting of Mn, ^{99m}Tc, ¹⁸⁶Re or ¹⁸⁸Re in the form of a permetallate solution.

14. A kit for the preparation of a diagnostic or therapeutic pharmaceutical composition, comprising (i) a suitable substrate to be labelled with a metal selected from the group consisting of Mn, ^{99m}Tc, ¹⁸⁶Re or ¹⁸⁸Re, (ii) a reducing agent soluble in water but not substantially decomposed by water, (iii) a base, (iv) if desired, a stabilizing agent and/or a chelator, (v) if desired one or more inert pharmaceutically acceptable carriers and/or formulating agents and/or adjuvants, at least one of said ingredients (i) to (v) being stored in a container having an atmosphere containing a sufficient amount of carbon monoxide, said ingredients (i) to (v) optionally independently being combined, and (vi) instructions for use with a prescription for reacting the ingredients of the kit with said metal in the form of a permetallate solution.

## Patentansprüche

1. Verfahren zur Herstellung einer Verbindung der allgemeinen Formel
*fac*-[M(CO)₃(OH₂)₃]⁺ (I),
worin M für Mn, ^{99m}Tc, ¹⁸⁶Re oder ¹⁸⁸Re steht,
durch Umsetzen eines Metalls in der Permetallatform mit Kohlenmonoxid und einem Reduktionsmittel,
**dadurch gekennzeichnet,**
**dass** ein Gemisch aus einer Base, einem wasserlöslichen, aber nicht wesentlich durch Wasser dissoziierten Reduktionsmittel, und wahlweise einem Stabilisator in einem wasserhaltigen Lösungsmittelsystem, das eine Lösung des Metalls in der Permanganat-, Pertechnetat- oder Perrhenatform enthält, in Gegenwart von Kohlenmonoxid gelöst wird.

2. Verfahren zur Herstellung einer Verbindung der allgemeinen Formel
*fac*-[M(CO)₃(X)₂L₁]ⁿ (II),
*fac*-[M(CO)₃(X)L₂]ⁿ (III)
oder
*fac*-[M(CO)₃L₃]ⁿ (IV),
worin:
M für Mn, ^{99m}Tc, ¹⁸⁶Re oder ¹⁸⁸Re steht,
L₁ für einen einzähnigen Liganden steht,
L₂ aus der Gruppe bestehend aus einem zweizähnigen Liganden und zwei einzähnigen Liganden ausgewählt ist;
L₃ aus der Gruppe bestehend aus einem dreizähnigen Liganden, einem einzähnigen Liganden und einem zweizähnigen Liganden, und drei einzähnigen Liganden ausgewählt ist;
X für H₂O oder einem Halogenidion steht;
n für die Summe der Ladungen der Liganden L₁ oder L₂ oder L₃ und X, erhöht um eine +-Ladung, steht;
**dadurch gekennzeichnet,**
**dass** eine Verbindung der allgemeinen Formel (I), hergestellt nach Anspruch 1, mit den Liganden L₁ oder L₂ oder L₃, wahlweise in Gegenwart eines Halogenids, umgesetzt wird.

3. Verfahren zur Herstellung einer Verbindung der allgemeinen Formel
*fac*-[M(CO)₃(X)₂L₁]ⁿ (II),
*fac*-[M(CO)₃(X)L₂]ⁿ (III)
oder
*fac*-[M(CO)₃L₃]ⁿ (IV),
worin:
M für Mn, ^{99m}Tc, ¹⁸⁶Re oder ¹⁸⁸Re steht,
L₁ für einen einzähnigen Liganden steht,
L₂ aus der Gruppe bestehend aus einem zweizähnigen Liganden und zwei einzähnigen Liganden ausgewählt ist;
L₃ aus der Gruppe bestehend aus einem dreizähnigen Liganden, einem einzähnigen Liganden und einem zweizähnigen Liganden, und drei einzähnigen Liganden ausgewählt ist;
X für H₂O oder einem Halogenidion steht;
n für die Summe der Ladungen der Liganden L₁ oder L₂ oder L₃ und X, erhöht um eine +-Ladung, steht;
**dadurch gekennzeichnet,**
**dass** das Gemisch aus einer Base, den Liganden L₁ oder L₂ oder L₃, einem wasserlöslichen, aber nicht wesentlich durch Wasser dissoziierten Reduktionsmittel und wahlweise einem Stabilisator in einem wasserhaltigen Lösungsmittelsystem, das eine Lösung des Metalls in der Permanganat-, Pertechnetat- oder Perrhenatform enthält, in Gegenwart von Kohlenmonoxid und wahlweise in Gegenwart eines Halogenids gelöst wird.

4. Verfahren nach Anspruch 2 und 3, **dadurch gekennzeichnet, dass** die Liganden L₁-L₃ biologisch wirksame Substanzen sind oder davon abstammen, die aus der Gruppe aus Aminosäuren, Peptiden, Proteinen, Zucker, kleine Rezeptorbindungsmoleküle und Körperzellen ausgewählt sind.

5. Verfahren zur Markierung nach Anspruch 4, **dadurch gekennzeichnet, dass** das Substrat eine Aminosäure, ein Peptid oder ein Protein ist.

6. Verfahren nach Anspruch 1 bis 3, **dadurch gekennzeichnet, dass** die Base eine anorganische Base ist, vorzugsweise ausgewählt aus der Gruppe aus NaOH, KOH, NaHCO₃, Na₂CO₃, KHCO₃, K₂CO₃, Ca(OH)₂ und Mg(OH)₂.

7. Verfahren nach Anspruch 1, 3 oder 6, **dadurch gekennzeichnet, dass** das Molverhältnis von der Base zu dem Reduktionsmittel zwischen 0,1 und 2, vorzugsweise etwa 0,35 ist.

8. Verfahren nach Anspruch 1, 3 oder 6 -7, **dadurch gekennzeichnet, dass** das Reduktionsmittel aus der Gruppe aus Borhydridanion und substituierten Borhydridanion, wobei bis zu drei der Wasserstoffatome, die das Borhydridanion enthält, durch inerte Substituenten ersetzt wurden, ausgewählt ist.

9. Verfahren nach Anspruch 8, **dadurch gekennzeichnet, dass** das Reduktionsmittel ein Borhydridanion ist, das von einem Salz, ausgewählt aus der Gruppe aus Natriumborhydrid, Kaliumborhydrid, Lithiumborhydrid und Zinkborhydrid, abstammt.

10. Verfahren nach Anspruch 9, **dadurch gekennzeichnet, dass** das Reduktionsmittel Natriumborhydrid ist.

11. Verfahren nach Anspruch 10, **dadurch gekennzeichnet, dass** das Molverhältnis von Reduktionsmittel zu Permetallat mindestens 3 beträgt.

12. Verfahren nach Anspruch 1, 3 und 6-11, **dadurch gekennzeichnet, dass** die Reaktionstemperatur zwischen 20°C und 100°C liegt und vorzugsweise etwa 75°C beträgt.

13. Kit zur Herstellung einer Markierungszusammensetzung, umfassend (i) ein wasserlösliches, aber nicht wesentlich durch Wasser dissoziiertes Reduktionsmittel, (ii) eine Base, (iii) gegebenenfalls einen Stabilisator und/oder Chelatbildner und (iv) gegebenenfalls ein oder mehrere inerte pharmazeutisch annehmbare Träger und/oder Formulierungsmittel und/oder Hilfsmittel, wobei mindestens eine der Komponenten (i) bis (iv) in einem Behälter mit einer Atmosphäre gelagert wird, die eine ausreichende Menge Kohlenmonoxid enthält, und die Komponenten (i) bis (iv) gegebenenfalls unabhängig kombiniert werden können, und (v) eine Gebrauchsanweisung mit einer Anleitung zur Umsetzung der Kit-Komponenten mit einem Metall, ausgewählt aus der Gruppe aus Mn, ^{99m}Tc, ¹⁸⁶Re oder ¹⁸⁸Re in Form einer Permetallat-Lösung.

14. Kit zur Herstellung einer diagnostischen oder therapeutischen pharmazeutischen Zusammensetzung, umfassend (i) ein geeignetes Substrat zur Markierung mit einem Metall, ausgewählt aus der Gruppe aus Mn, ^{99m}Tc, ¹⁸⁶Re oder ¹⁸⁸Re, (ii) ein wasserlösliches, aber nicht wesentlich durch Wasser dissoziiertes Reduktionsmittel, (iii) eine Base, (iv) gegebenenfalls einen Stabilisator und/oder Chelatbildner und (v) gegebenenfalls ein oder mehrere inerte pharmazeutisch annehmbare Träger und/oder Formulierungsmittel und/oder Hilfsmittel, wobei mindestens eine der Komponenten (i) bis (v) in einem Behälter mit einer Atmosphäre gelagert wird, die eine ausreichende Menge Kohlenmonoxid enthält, und die Komponenten (i) bis (v) gegebenenfalls unabhängig kombiniert werden können, und (vi) eine Gebrauchsanweisung mit einer Anleitung zur Umsetzung der Kit-Komponenten mit dem Metall in Form einer Permetallat-Lösung.

## Revendications

1. Procédé de préparation d'un composé de formule générale
*fac*-[M(CO)₃(OH₂)₃]⁺ (I)
dans laquelle M est Mn, ^{99m}Tc, ¹⁸⁶Re ou ¹⁸⁸Re,
qui consiste à faire réagir un métal sous forme de permétallate avec du monoxyde de carbone et un agent réducteur, **caractérisé en ce que** l'on dissout un mélange d'une base, d'un agent réducteur soluble dans l'eau mais pas sensiblement décomposé par l'eau et éventuellement d'un agent stabilisant, dans une eau contenant un système de solvant contenant une solution du métal sous forme de permanganate, de pertechnétate ou de perrhénate, en présence de monoxyde de carbone.

2. Procédé de préparation d'un composé de formule générale
*fac*-[M(CO)₃(X)₂L₁]ⁿ (II),
*fac*-[M(CO)₃(X)L₂]ⁿ (III)
ou
*fac*-[M(CO)₃L₃]ⁿ (IV),
formules dans lesquelles:
M est Mn, ^{99m}Tc, ¹⁸⁶Re ou ¹⁸⁸Re;
L₁ est un ligand monodenté,
L₂ est choisi dans le groupe constitué par un ligand bidenté et deux ligands monodentés, et
L3 est choisi dans le groupe constitué par un ligand tridenté, un ligand monodenté et un ligand bidenté, et trois ligands monodentés;
X est H₂O ou un ion halogénure;
n est la somme de la charge des ligands L₁ ou L₂ ou L₃ et X, augmenté d'une charge +;
**caractérisé en ce que** l'on fait réagir un composé de formule générale I préparé selon la revendication 1 avec les ligands L₁ ou L₂ ou L₃, éventuellement en présence d'un halogénure.

3. Procédé de préparation d'un composé de formule générale
*fac*-[M(CO)₃(X)₂L₁]ⁿ (II),
*fac*-[M(CO)₃(X)L₂]ⁿ (III)
ou
*fac*-[M(CO)₃L₃]ⁿ (IV),
formules dans lesquelles:
M est Mn, ^{99m}Tc, ¹⁸⁶Re ou ¹⁸⁸Re;
L₁ est un ligand monodenté,
L₂ est choisi dans le groupe constitué par un ligand bidenté et deux ligands monodentés, et
L3 est choisi dans le groupe constitué par un ligand tridenté, un ligand monodenté et un ligand bidenté, et trois ligands monodentés;
X est H₂O ou un ion halogénure;
n est la somme de la charge des ligands L₁ ou L₂ ou L₃ et X, augmenté d'une charge +;
**caractérisé en ce que** l'on dissout un mélange d'une base, des ligands L₁ ou L₂ ou L₃, d'un agent réducteur soluble dans l'eau mais pas sensiblement décomposé par l'eau et éventuellement d'un agent stabilisant, dans une eau contenant un système de solvant contenant une solution du métal sous forme de permanganate, de pertechnétate ou de perrhénate, en présence de monoxyde de carbone et éventuellement en présence d'un halogénure.

4. Procédé selon les revendications 2-3, **caractérisé en ce que** les ligands L₁-L₃ sont des substrats ayant une activité biologique choisis dans le groupe constitué par les acides aminés, les peptides, les protéines, les sucres, les petites molécules de liaison aux récepteurs et les cellules ou sont dérivés de ces substrats.

5. Procédé de marquage selon la revendication 4, **caractérisé en ce que** le substrat est un acide aminé, un peptide ou une protéine.

6. Procédé selon la revendication 1 ou 3, **caractérisé en ce que** la base est une base minérale, choisie de préférence dans le groupe constitué par NaOH, KOH, NaHCO₃, Na₂CO₃, KHCO₃, K₂CO₃, Ca(OH)₂ et Mg(OH)₂.

7. Procédé selon la revendication 1, 3 ou 6, **caractérisé en ce que** le rapport molaire entre la base et l'agent réducteur est compris entre 0,1 et 2, et est de préférence d'environ 0,35.

8. Procédé selon la revendication 1, 3 ou 6-7, **caractérisé en ce que** l'agent réducteur est choisi dans le groupe constitué par l'anion borohydrure et un anion borohydrure substitué dans lequel jusqu'à trois des atomes d'hydrogène constituant l'anion borohydrure ont été remplacés par des substituants inertes.

9. Procédé selon la revendication 8, **caractérisé en ce que** l'agent réducteur est un anion borohydrure dérivé d'un sel choisi dans le groupe constitué par le borohydrure de sodium, le borohydrure de potassium, le borohydrure de lithium et le borohydrure de zinc.

10. Procédé selon la revendication 9, **caractérisé en ce que** l'agent réducteur est le borohydrure de sodium.

11. Procédé selon la revendication 10, **caractérisé en ce que** le rapport molaire de l'agent réducteur au permétallate est d'au moins 3.

12. Procédé selon les revendications 1, 3 et 6-11, **caractérisé en ce que** la température de la réaction est comprise entre 20°C et 100°C et est de préférence d'environ 75°C.

13. Trousse pour la préparation d'une composition de marquage, comprenant (i) un agent réducteur soluble dans l'eau mais pas sensiblement décomposé par l'eau, (ii) une base, (iii) le cas échéant, un agent stabilisant et/ou un chélateur et (iv), le cas échéant, un ou plusieurs véhicules et/ou agents de formulation et/ou adjuvants inertes, pharmaceutiquement acceptables, au moins un desdits ingrédients (i) à (iv) étant conservé dans un récipient dont l'atmosphère contient une quantité suffisante de monoxyde de carbone, lesdits ingrédients (i) à (iv) étant indépendamment combinés, et (v) un mode d'emploi avec une prescription pour faire réagir les ingrédients de la trousse avec un métal choisi dans le groupe constitué par Mn, ^{99m}Tc, ¹⁸⁶Re ou ¹⁸⁸Re sous forme d'une solution de permétallate.

14. Trousse pour la préparation d'une composition pharmaceutique diagnostique ou thérapeutique, comprenant (i) un substrat approprié à marquer avec un métal choisi dans le groupe constitué par Mn, ^{99m}Tc, ¹⁸⁶Re ou ¹⁸⁸Re, (ii) un agent réducteur soluble dans l'eau mais pas sensiblement décomposé par l'eau, (iii) une base, (iv) le cas échéant, un agent stabilisant et/ou un chélateur, (v) le cas échéant, un ou plusieurs véhicules et/ou agents de formulation et/ou adjuvants inertes, pharmaceutiquement acceptables, au moins un desdits ingrédients (i) à (v) étant conservé dans un récipient dont l'atmosphère contient une quantité suffisante de monoxyde de carbone, lesdits ingrédients (i) à (v) étant indépendamment combinés, et (vi) un mode d'emploi avec une prescription pour faire réagir les ingrédients de la trousse avec ledit métal sous forme d'une solution de permétallate.
